(19) Europäisches Patentamt
European Patent Office
Office européen des brevets

(11) EP 1 680 421 B1

(12) **FASCICULE DE BREVET EUROPEEN**

(45) Date de publication et mention de la délivrance du brevet:
**13.02.2008 Bulletin 2008/07**

(21) Numéro de dépôt: **04791554.1**

(22) Date de dépôt: **15.10.2004**

(51) Int Cl.:
*C07D 471/08* (2006.01)

(86) Numéro de dépôt international:
**PCT/FR2004/002643**

(87) Numéro de publication internationale:
**WO 2005/037783 (28.04.2005 Gazette 2005/17)**

(54) **COMPOSES TRICYCLIQUES COMME INHIBITEURS DE TRANSPORT DE GLYCINE**

VERWENDUNG TRICYCLISCHER VERBINDUNGEN ALS INHIBITOREN DES GLYCINTRANSPORTS

USE OF TRICYCLIC COMPOUNDS AS GLYCINE TRANSPORT INHIBITORS

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **17.10.2003 FR 0312165**

(43) Date de publication de la demande:
**19.07.2006 Bulletin 2006/29**

(73) Titulaire: **Sanofi-Aventis**
**75013 Paris (FR)**

(72) Inventeurs:
- **DARGAZANLI, Gihad**
**F-94230 Cachan (FR)**
- **ESTENNE-BOUHTOU, Geneviève**
**F-94550 Chevilly-Larue (FR)**
- **MAGAT, Pascale**
**F-91380 Chilly-Mazarin (FR)**
- **MARABOUT, Benoît**
**F-91300 Massy (FR)**
- **ROGER, Pierre**
**F-78180 Montigny le Bretonneux (FR)**

(74) Mandataire: **Monain, Patrice et al**
**Sanofi-Aventis**
**Departement Brevets**
**174, avenue de France**
**75013 Paris (FR)**

(56) Documents cités:
**WO-A-99/45011**

- **CAULFIELD W L ET AL: "The first potent and selective Inhibitors of the Glycine Transporter Type 2" JOURNAL OF MEDICINAL CHEMISTRY, AMERICAN CHEMICAL SOCIETY. WASHINGTON, US, vol. 44, no. 17, 16 août 2001 (2001-08-16), pages 2679-2682, XP002183670 ISSN: 0022-2623**
- **GALLARDO M.A. ET AL.: "Influence of hypo-osmolality on the activity of short-chain neutral amino acid carriers in trout red blood cells" J. MEMBRANE BIOL., vol. 155, 1997, pages 113-119, XP002286400**
- **BRUNNER H.ET AL.: "alpha-Amino acid derivatives by enantioselective decarboxylation" EUR. J. ORG. CHEM., 1 août 2003 (2003-08-01), pages 2854-2862, XP002286401**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 680 421 B1

## Description

La présente invention a pour objet des dérivés de *N*-hétérocyclylméthylbenzamides, leur préparation et leur application en thérapeutique. WO 99/45011 montre des composés tricycliques qui présentent une activité comme inhibiteurs des transporteurs de la glycine.

Les composés de l'invention répondent à la formule générale (I)

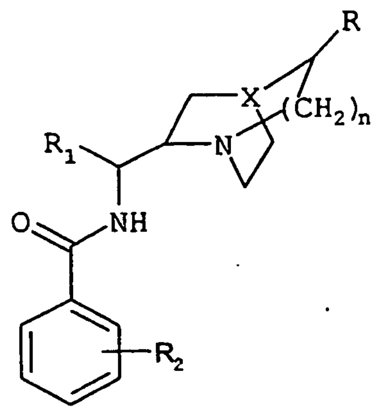

(I)

dans laquelle

R représente un atome d'hydrogène ou un groupe vinyle ;
n représente 0 ou 1 ou 2 quand R représente un atome d'hydrogène et n représente 1 quand R représente un groupe vinyle ;
X représente un groupe de formule CH ou un atome d'azote quand R représente un atome d'hydrogène et X représente un groupe de formule CH quand R représente un groupe vinyle ;
$R_1$ représente soit un groupe phényle ou naphtyle éventuellement substitué par un ou plusieurs substituants choisis parmi les atomes d'halogène, les groupes $(C_1-C_6)$alkyle, hydroxy et $(C_1-C_6)$alcoxy linéaires ou ramifiés, le groupe trifluorométhyle, soit un groupe cyclohexyle, soit un groupe hétéroaryle choisi parmi les groupes thiényle, pyridinyle, oxazolyle, furanyle, thiazolyle, quinoléinyle et is oquinoléinyle ;
$R_2$ représente soit un atome d'hydrogène, soit un ou plusieurs substituants choisis parmi les atomes d'halogène et les groupes trifluorométhyle, $(C_1-C_6)$alkyle, $(C_1-C_6)$alcoxy, thiényle, phényloxy, hydroxy, mercapto, thio$(C_1-C_6)$alkyle, cyano ou un groupe de formule générale $-NR_4R_5$, $SO_2NR_4R_5$, $-SO_2-(C_1-C_6)$alkyle, - $SO_2$-phényle, $-CONR_4R_5$, $-COOR_7$, $-CO-(C_1-C_6)$alkyle, -CO-phényle, - $NHCOR_8$, $-NHSO_2-(C_1-C_6)$ alkyle, $-NHSO_2$-phényle et $-NHSO_2NR_4R_5$ ou un groupe de formule $-OCF_2O-$ lié aux positions 2 et 3 du groupe phényle ;
les groupes $(C_1-C_s)$alkyle, $(C_1-C_6)$alcoxy, $-SO_2-(C_1-C_6)$alkyle, $-CO-(C_1-C_6)$ alkyle et $-NHSO_2-(C_1-C_6)$ alkyle étant éventuellement substitués par un ou plusieurs groupes $R_3$ ; les groupes phényle, $-SO_2$-phényle, -CO-phényle et -$NHSO_2$-phényle étant éventuellement substitués par un groupe $R_6$; $R_3$ représente un atome d'halogène, un groupe phényle, $(C_1-C_6)$alcoxy, $-NR_4R_5$ ;
$R_4$ et $R_5$ représentent indépendamment l'un de l'autre un atome d'hydrogène ou un groupe $(C_1-C_6)$alkyle ou $R_4$ et $R_5$ forment avec l'atome d'azote qui les porte un cycle pyrrolidine, un cycle pipéridine ou un cycle morpholine ;
$R_6$ représente un atome d'hydrogène, un atome d'halogène ,
un groupe trifluorométhyle,un groupe cyano, un groupe hydroxy, un groupe mercapto, un groupe $(C_1-C_6)$alkyle ou $(C_1-C_6)$alcoxy ; $R_7$ représente un atome d'hydrogène ou un groupe $(C_1-C_6)$alkyle éventuellement substitué par un ou plusieurs groupes $R_3$, ou un groupe phényle éventuellement substitué par un groupe $R_6$ ;
$R_8$ représente un groupe $(C_1-C_6)$alkyle éventuellement substitué par un ou plusieurs groupes $R_3$, ou un groupe $(C_1-C_6)$alcoxy, ou un groupe phényle éventuellement substitué par un groupe $R_6$.

Parmi les composés de formule générale (I), on distingue un certain nombre de sous ensembles de composés préférés :

Groupe 1 : les composés de configuration thréo et de formule générale (I) dans laquelle n représente 0 ou 1 ;

Groupe 2 : les composés du groupe 1 dans la formule desquels X représente un groupe de formule CH ;

Groupe 3 : les composés du groupe 2 dans la formule desquels R représente un atome d'hydrogène ;

Groupe 4 : les composés du groupe 3 dans la formule desquels n représente 1 ;

Groupe 5 : les composés du groupe 4 dans la formule desquels $R_1$ représente un groupe phényle éventuellement substitué.

Les composés de formule (I) peuvent comporter plusieurs centres asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Plus particulièrement, les composés de formule (I), pour lesquels R = H, peuvent exister sous forme de diastéréoisomères thréo ((1*S*, 2*S*) et (1*R*, 2*R*)) ou érythro ((1*S*, 2*R*) et (1*R*, 2*S*)) ou d'énantiomères purs ou en mélange de tels isomères .

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Les composés de l'invention présentent une activité particulière comme inhibiteurs spécifiques des transporteurs de la glycine glyt1 et/ou glyt2.

Les composés de formule générale (I) peuvent être préparés par un procédé illustré par le schéma 1 qui suit.

Schéma 1

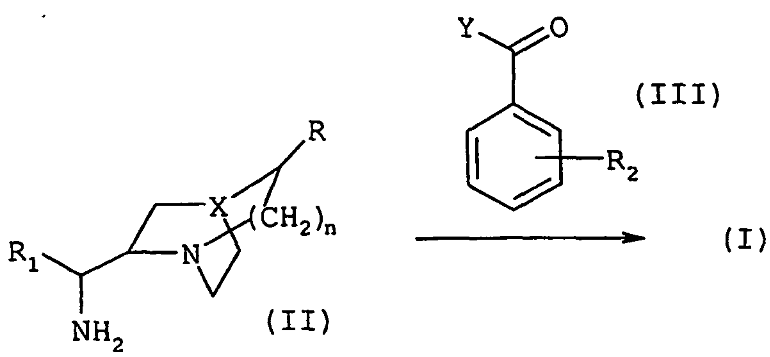

Selon le schéma 1, on effectue un couplage d'une diamine de formule générale (II), dans laquelle n, X, R et $R_1$ sont tels que définis ci-dessus, avec un acide activé ou un chlorure d'acide de formule générale (III) dans laquelle Y représente un groupe nucléofuge, tel qu'un atome d'halogène, et $R_2$ est tel que défini ci-dessus, en utilisant les méthodes connues de l'homme du métier.

Les diamines de formule générale (II), dans laquelle R = H et n, X et $R_1$ sont tels que définis ci-dessus, peuvent être préparées par un procédé illustré par le schéma 2 qui suit.

Schéma 2

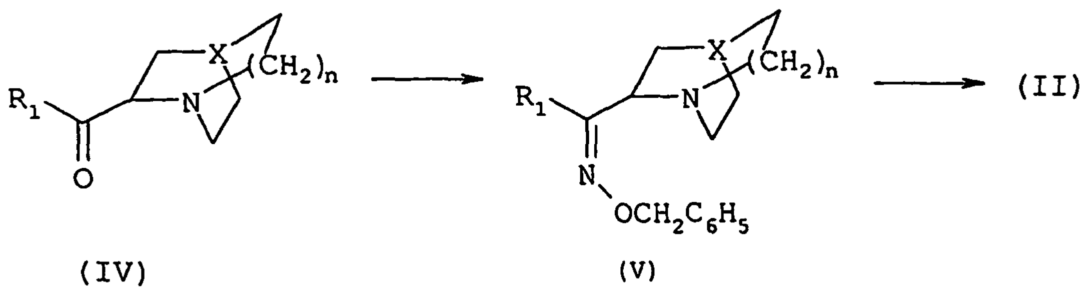

On fait réagir la cétone de formule générale (IV), dans laquelle n, X et $R_1$ sont tels que définis ci-dessus, avec le chlorhydrate de benzyloxyhydroxylamine, au reflux de la pyridine, pour obtenir l'oxime de formule générale (V).

Les deux formes *Z* et *E* de l'oxime peuvent être séparées selon des méthodes connues de l'homme du métier telle que la chromatographie sur colonne de gel de silice.

L'oxime (V) de préférence sous forme de *Z* chlorhydrate est ensuite réduite au reflux du tétrahydrofuranne par l'hydrure double d'aluminium et de lithium pour fournir la diamine de formule générale (II) majoritairement thréo.

Par réduction de la forme *E* de l'oxime de formule générale (V), on obtient un mélange de diamine (II) sous forme des deux diastéroisomères (thréo/érythro).

Les diastéréoisomères érythro et thréo peuvent être séparés selon des méthodes connues de l'homme du métier telle que la chromatographie sur colonne de gel de silice.

Une autre variante de préparation des diamines de formule générale (II), dans laquelle R et $R_1$ sont tels que définis ci-dessus, n est égal à 1 et X est un CH, est illustrée par le schéma 3 qui suit.

Schéma 3

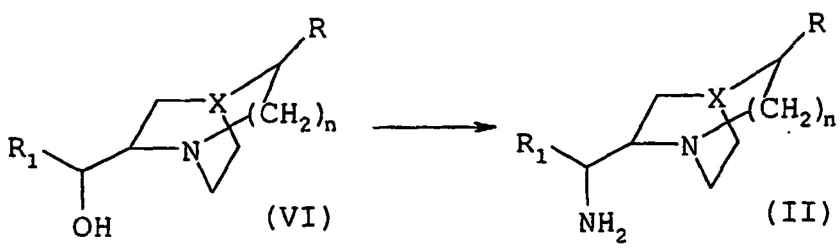

Les alcools de formule générale (VI) sont transformés en amines par une réaction de Mitsunobu selon la méthode décrite dans Bull.Soc.Chim.Belg. (106), 1997, 77-84 et dans Tetrahedron : Asymmetry, (6), 1995,1699-1702.

Par ailleurs les composés chiraux de formule générale (I), correspondant aux énantiomères (1*R*,2*R*) ou (1*S*, 2*S*) du diastéréoisomère thréo et aux énantiomères (1*S*, 2*R*) ou (1*R*, 2*S*) du diastéréoisomère érythro, peuvent être également obtenus soit par séparation des composés racémiques par chromatographie liquide à haute performance (CLHP) sur colonne chirale, soit à partir de l'amine chirale obtenue soit par dédoublement de l'amine racémique de formule générale (II), par utilisation d'un acide chiral, tel que l'acide tartrique, l'acide camphorsulfonique, l'acide dibenzoyltartrique, la N-acétylleucine, par la recristallisation fractionnée et préférentielle d'un sel diastéréoisomérique dans un solvant de type alcool, soit par une synthèse énantiosélective à partir d'un alcool chiral érythro ou thréo en utilisant un procédé analogue à celui décrit dans le schéma 3. Les alcools chiraux peuvent être obtenus par une méthode analogue à celle décrite dans Tetrahedron, (55), 1999,2795-2810. Dans le cas où R représente un groupe vinyle et $R_1$ représente un groupe quinoléinyle, la diamine de formule générale (II) peut être préparée selon le schéma 3 en utilisant les alcools chiraux commerciaux correspondants.

La cétone de formule générale (IV) racémique peut être préparée soit par déprotonation d'un complexe activé des amines cycliques pontées et réaction avec un électrophile, tel qu'un ester ou un amide de Weinreb, selon une méthode analogue à celle décrite dans Chem.Commun., 1999, 1927-1928, soit par réaction d'un organométallique sur

l'ester éthylique de l'acide 2-quinuclidinique , selon une méthode analogue à celle décrite dans J. Med. Chem., 1980, 180-184, soit par oxydation de l'alcool correspondant obtenu par différentes méthodes analogues à celles décrites dans J. Org. Chem., 50, 1985, 29-31 et Chem. Comm., 1999, 1927-1929 avec des oxydants connus de l'homme du métier tels que le dioxyde de manganèse ou le système chlorure d'oxalyle-diméthylsulfoxyde.
Les alcools de formule générale (VI) peuvent être également obtenus par réduction des cétones de formule générale (IV) correspondantes dans des conditions connues de l'homme du métier.

Les acides et chlorures d'acide de formule générale (III) sont disponibles dans le commerce ou préparés par analogie à des méthodes connues de l'homme du métier.

Par exemple, l'acide 4-amino-3-chloro-5-trifluorométhyl-benzoïque peut être préparé par chloration de l'acide 4-amino-5-trifluorométhylbenzoïque avec le chlorure de sulfuryle dans un solvant chloré tel que le chloroforme, selon une méthode analogue à celle décrite dans Arzneim. Forsch., 34, 11a, (1984), 1668-1679

L'acide 2,6-dichloro-3-trifluorométhylbenzoïque peut être préparé par des méthodes analogues à celles décrites dans US3,823,134.

Les acides benzoïques dérivés de sulfonamides peuvent être préparés selon des méthodes analogues à celles décrites dans les brevets DE-2436263, BE-620741, DE-1158957, US-3112337, GB-915259, US-3203987, DE-642758, EP-68700, FR-2396757,

DE-2734270, et dans J. Pharm. Pharmacol. (1962), 14, 679-685. Les acides métachlorosulfonylés peuvent être obtenus selon une méthode analogue à celles décrites dans J. Chem. Soc. (C), (1968), 13, et dans les brevets US-2273444, DE-19929076, EP-0556674.

La chlorosulfonylation en position *ortho* ou *para* peut être réalisée à partir d'un sel de diazonium selon une méthode analogue à celle décrite dans le brevet US-3663615, avec l'acide 4-amino-3-chlorobenzoïque.

Les sulfonamides sont obtenus par réaction des dérivés chlorosulfonylés en présence d'un excès d'amine dans un solvant tel que le tétrahydrofuranne, à température ambiante ou au reflux.

Les sulfonamides secondaires peuvent être méthylés selon une méthode analogue à celle décrite dans le brevet BE-620741.

Les sulfonamides primaires peuvent être mis en réaction avec un isocyanate, dans un solvant tel que le tétrahydrofuranne, en présence d'une base telle que le carbonate de potassium. Certains dérivés sulfoxydes d'acides benzoïques sont décrits dans les brevets DE-2056912, DE-2901170 et US-3953476, ou peuvent être obtenus par des méthodes analoques à celles décrites dans le brevet BE-872585 et dans J. Org. Chem. ((1991), 56(1), 4976-4977.

Les dérivés d'acides benzoïques de formule générale (III), dans laquelle $R_2$ représente un groupe alkyle ramifié peuvent être préparés selon des méthodes analoques à celle décrites dans le brevet US-4879426 et dans Syn. Lett. (1996), 473-474 et J. Med. Chem. (2001), 44, 1085-1098.

Les dérivés d'acides benzoïques de type biphényle peuvent être préparés selon des méthodes connues de l'homme du métier. Enfin, les acides benzoïques carbonylés peuvent être synthétisés selon des méthodes analogues à celles décrites dans les brevets US-3725417 et GB-913100 et dans Chem. Pharm. Bull., (1988), 36(9), 3462-3467 et J. Labelled Compd. Radiopharm., (1997), 39(6), 501-508.

Les esters ou amides peuvent être introduits par carbonylation directe avec une base forte en para de l'acide, dans les conditions décrites dans Tetrahedron Lett., (2000), 41, 3157-3160.

Enfin les dérivés cyano des acides benzoïques sont obtenus par chauffage d'un acide ou ester benzoïque halogéné en présence de cyanure de potassium, d'un catalyseur du type palladiumtétrakis triphénylphosphine dans un solvant du type tétrahydrofuranne, selon une méthode analogue à celle décrite dans J. Org. Chem. (1967) 62, 25, 8634-8639.

D'autres acides et chlorures d'acides de formule générale (III) peuvent être obtenus selon des méthodes analogues à celles décrites dans les brevets EP-0556672, US-3801636, et dans J. Chem. Soc., (1927), 25, Chem. Pharm. Bull., (1992), 1789-1792, Aust. J. Chem., (1984), 1938-1950 et J. O. C., (1980), 527.

Les exemples qui vont suivre illustrent la préparation de quelques composés de l'invention. Les microanalyses élémentaires, et les spectres I.R. et R.M.N. et la CLHP sur colonne chirale confirment les structures et les puretés énantiométriques des composés obtenus.

Les numéros indiqués entre parenthèses dans les titres des exemples correspondent à ceux de la 1ère colonne du tableau donné plus loin.

Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

Exemple 1 (Composé N°3).

Chlorhydrate de thréo-2-chloro-*N*-[(1-azabicyclo[2.2.2]oct-2-yl)phénylméthyl]-3-trifluorométhylbenzamide 1:1.

1.1. Chlorhydrate de (*Z*)-1-azabicyclo[2.2.2.]oct-2-yl(phenyl)méthanone *O*-benzyloxime.

Dans un ballon de 100 ml muni d'une agitation magnétique, on introduit 2,2 g (9,35 mmoles) de 1-azabicyclo [2.2.2.]oct-2-yl(phényl)méthanone (Chem.Commun., 1999, 1927-1928) et 3 g (18,69 mmoles) de chlorhydrate de benzyloxyhydroxylamine dans 50 ml de pyridine, et on chauffe le mélange au reflux pendant 20 h.

Après évaporation des solvants sous pression réduite, on dilue le résidu avec de l'eau et du chloroforme , on sépare la phase aqueuse, et on l'extrait avec du chloroforme. Après lavage des phases organiques réunies, séchage sur sulfate de sodium et évaporation du solvant sous pression réduite, on purifie le résidu par chromatographie sur colonne de gel de silice en éluant avec un mélange de chloroforme et de méthanol.

On obtient 0,5 g d'une fraction correspondant à la (*E*)-1-azabicyclo[2.2.2.]oct-2-yl(phényl)méthanone *O*-benzyloxime et 2,25 g d'une autre fraction correspondant au chlorhydrate de la (*Z*)-1-azabicyclo[2.2.2.]oct-2-yl(phényl) méthanone *O*-benzyloxime PF 195-197°C.

1.2. thréo-[1-azabicyclo[2.2.2.]oct-2-yl(phényl)méthyl]amine

Dans un tricol de 250 ml muni d'une agitation magnétique, sous atmosphère d'azote, on place 1,3 g (34,32 mmoles) d'hydrure double d'aluminium et de lithium en suspension dans 10 ml de tétrahydrofuranne, on ajoute, par portions, 2,2 g(6,16 mmoles) de chlorhydrate de (*Z*)-1-azabicyclo[2.2.2.]oct-2-yl(phényl)méthanone *O*-benzyloxime et on chauffe au reflux pendant 2 h.

Après refroidissement on hydrolyse la solution à 0°C par successivement 1,3 ml d'eau puis 1,3 ml de soude aqueuse à 15% et 3,9 ml d'eau. On filtre le mélange hétérogène sur célite®, on concentre le filtrat sous pression réduite puis on dilue le résidu avec de l'acide chlorhydrique 1N et du chloroforme. On sépare la phase organique et on basifie la phase aqueuse avec de l'ammoniaque. On l'extrait 2 fois avec du chloroforme.

Après lavage des phases organiques réunies, séchage sur sulfate de sodium et évaporation du solvant sous pression réduite, on obtient 1,25 g de thréo-[1-azabicyclo[2.2.2.]oct-2-yl(phényl)méthyl]amine sous forme d'une huile qui cristallise et qu'on utilise telle quelle dans l'étape suivante.
Point de fusion : 120-140°C.

1.3. Chlorhydrate de thréo-2-chloro-N-[(1-aza-bicyclo[2.2.2]oct-2-yl)phénylméthyl]-3-trifluorométhylbenzamide 1:1.

Dans un ballon de 100 ml muni d'une agitation magnétique, on place 0,51 g (2,12 mmoles) de chlorure d'acide 2-chloro-3-trifluorométhylbenzoïque en solution dans 5 ml de chloroforme en présence de 0,29 g (2,12 mmoles) de carbonate de potassium à 0°C et on verse une solution 0,42 g (1,93 mmole)de thréo-[1-azabicyclo[2.2.2.]oct-2-yl(phényl) méthyl]amine en solution dans 5 ml de chloroforme et on agite le mélange à température ambiante pendant 6 h.

Après hydrolyse à l'eau et dilution au chloroforme on sépare la phase aqueuse, et on l'extrait avec du chloroforme. Après lavage des phases organiques réunies, séchage sur sulfate de sodium et évaporation du solvant sous pression réduite, on purifie le résidu par chromatographie sur colonne de gel de silice en éluant avec un mélange de chloroforme et de méthanol. On obtient 0,18 g de produit huileux.

On dissout ce dernier dans quelques ml de propan-2-ol, on ajoute 6 ml d'une solution 0,1N d'acide chlorhydrique dans le propan-2-ol, et on concentre le mélange sous pression réduite afin de réduire le volume du solvant. Après trituration on isole finalement 0,15 g de chlorhydrate sous forme d'un solide.
Point de fusion: 257-262°C.

Exemple 2 (Composé N°4).

Chlorhydrate de thréo-2,6-dichloro-*N*-[(1-azabicyclo[2.2.2]oct-2-yl)phénylméthyl]-3-trifluorométhylbenzamide 1:1.

Dans un ballon de 100 ml muni d'une agitation magnétique on introduit 0,36 g (1,38 mmole) d'acide 2,6-dichloro-3-trifluorométhylbenzoïque, 0,187 g (1,38 mmole) d'hydroxybenzotriazole, 0,264 g (1,38 mmole)de chlorhydrate de 1-[3-(diméthylamino)propyl]-3-éthylcarbodiimide en solution dans 7 ml de chloroforme et on agite le mélange à température ambiante pendant 30 min.

On ajoute 0,3 g(1,38 mmole) de thréo-[1-azabicyclo[2.2.2.]oct-2-yl(phényl)méthyl]amine en solution dans 5 ml de chloroforme et on agite à température ambiante pendant une nuit.

Après hydrolyse à l'eau et dilution au chloroforme on sépare la phase aqueuse, et on l'extrait avec du chloroforme. Après lavage des phases organiques réunies, séchage sur sulfate de sodium et évaporation du solvant sous pression réduite, on purifie le résidu par chromatographie sur colonne de gel de silice en éluant avec un mélange de chloroforme et de méthanol. On obtient 0,37 g de produit huileux.

On dissout ce dernier dans quelques ml de propan-2-ol, on ajoute 20 ml d'une solution 0,1N d'acide chlorhydrique dans le propan-2-ol, et on concentre le mélange sous pression réduite afin de réduire le volume du solvant. Après trituration on isole finalement 0,35 g de chlorhydrate sous forme d'un solide.
Point de fusion : 270-273°C.

Exemple 3 (Composé N°14).

Chlorhydrate de 2-chloro-N-(8α,9*S*-cinchonan-9-yl)-3-trifluorométhylbenzamide 2:1.

3.1. 8α,9*S*-cinchonan-9-amine

Dans un tricol de 100 ml muni d'une agitation magnétique sous atmosphère d'azote on introduit 0,74 g (2,5 mmoles) de 8α,9*R*-cinchonan-9-ol (cinchonidine) et 0,79 g (3 mmoles)de triphénylphosphine en suspension dans 15 ml de tétrahydrofuranne, et on ajoute 3,5 ml d'une solution 0,9 M dans le benzène (3 mmoles) d'acide hydrazoïque. A cette solution on ajoute, goutte à goutte, une solution de 0,55 ml (2,75 mmoles) de diisopropylcarbodiimide dans 1,5 ml de tétrahydrofuranne et on chauffe à 40°C pendant 16 h. On ajoute 0,65 g(2,5 mmoles) de triphénylphosphine et on agite pendant 30 min, on ajoute 0,5 ml d'eau et on reprend l'agitation pendant 6 h.

On hydrolyse avec de l'acide chlorhydrique 1 N et on dilue avec du chloroforme. On basifie la phase aqueuse avec de l'ammoniaque et on l'extrait plusieurs fois avec du chloroforme. Après lavage des phases organiques réunies, séchage sur sulfate de sodium et évaporation du solvant sous pression réduite, on obtient 0,97 g d'une huile orangée contenant la 8α,9*S*-cinchonan-9-amine que l'on utilise brute dans l'étape suivante.

3.2. Chlorhydrate de 2-chloro-*N*-(8α,9*S*-cinchonan-9-yl)-3-trifluorométhylbenzamide 2:1.

Selon la méthode décrite dans l'exemple 1.3, en partant de 0,97 g (3,3 mmoles) de 8α,9*S*-cinchonan-9-amine, 0,84 g (3,4 mmoles) de chlorure d'acide 2-chloro-3-trifluorométhylbenzoïque et de 0,5 g (3,63 mmoles) de carbonate de potassium, on obtient 0,360 g d'huile qu'on dissout dans 30 ml d'acide chlorhydrique 1 N. On extrait la phase aqueuse au chloroforme puis on évapore le solvant sous pression réduite. On obtient ainsi 0,26 g de chlorhydrate sous forme d'un solide blanc
Point de fusion: 185-205°C ; $[\alpha]_D^{25}$= -5,4 (c=0,986, MeOH).

Exemple 4 (Composé N°17).

Chlorhydrate de 2,6-dichloro-*N*-[(1S)-[(2S) (1-azabicyclo[2.2.2]oct-2-yl)phénylméthyl]-3-(trifluorométhyl)benzamide 1:1

4.1 D-tartrate de (1*S*)-[(2*S*)-1-azabicyclo[2.2.2.]oct-2-yl(phényl)méthyl]amine.

On dissout 9,4 g (43,45 mmoles) de thréo-[1-azabicyclo[2.2.2.]oct-2-yl(phényl)méthyl]amine dans 150 ml d'éthanol. On verse une solution de 6,52 g(43,45 mmoles) d'acide D-tartrique en solution dans 200 ml d'éthanol. Après évaporation du solvant sous pression réduite, le résidu est placé dans 500 ml d'une solution d'éthanol et d'eau (9/1) puis chauffé jusqu'à dissolution. Après 3 recristallisations successives, on obtient 5,39 g de D-tartrate de (1*S*)-[(2*S*)-1-azabicyclo[2.2.2.]oct-2-yl(phényl)méthyl]amine.
Point de fusion: 125-135°C.
$[\alpha]_D^{25}$=-46.1 (c=0,616 ; MeOH).

4.2. Chlorhydrate de 2,6-dichloro-*N*-[(1*S*)-[(2*S*) (1-azabicyclo[2.2.2]oct-2-yl)phénylméthyl]-3-(trifluorométhyl)benzamide 1:1.

Dans un ballon de 100 ml muni d'une agitation magnétique, on place 3,33 g (12,02 mmoles) de chlorure d'acide 2,6-dichloro-3-(trifluorométhyl)benzoïque en solution dans 30 ml de chloroforme en présence de 1,82 g (13,22 mmoles) de carbonate de potassium à 0°C et on verse une solution de 2,6 g (12,02 mmoles) de (1*S*)-[(2*S*)-1-azabicyclo[2.2.2.]oct-2-yl(phényl)-méthyl]amine (obtenue par basification du sel décrit en 4.1 puis extraction) en solution dans 40 ml de chloroforme et on agite le mélange à température ambiante pendant 6 h.

Après hydrolyse à l'eau et dilution au chloroforme on sépare la phase aqueuse, et on l'extrait avec du chloroforme. Après lavage des phases organiques réunies, séchage sur sulfate de sodium et évaporation du solvant sous

pression réduite, on purifie le résidu par chromatographie sur colonne de gel de silice en éluant avec un mélange de chloroforme et de méthanol. On obtient 5,4 g de produit huileux.

On dissout ce dernier dans quelques ml de chloroforme, on ajoute 600 ml d'une solution d'éther saturé en acide chlorhydrique, et on concentre le mélange sous pression réduite.Le résidu est recristallisé dans l'acétate d'éthyle. On obtient ainsi 4,7 g de chlorhydrate de 2,6-dichloro-*N*-[(1*S*)-[(2*S*) (1-azabicyclo[2.2.2]oct-2-yl)phénylméthyl]-3-(trifluorométhyl)benzamide
Point de fusion : 264-268°C.
$[\alpha]_D^{25}$=+61, 1° (c=0, 32 ; MeOH)

Exemple 5 (Composé N°26).

Chlorhydrate de thréo *N*-[1-azabicyclo[2.2.2]oct-2-yl(4-fluorophenyl)methyl]-2,6-dichloro-3-(trifluoromethyl)benzamide 1:1.

5.1 1-azabicyclo[2.2.2]oct-2-yl(4-fluorophényl)méthanol.

Dans un tricol de 100 ml sous argon on place 1,11 g (10 mmoles) de quinuclidine dans 40 ml de tétrahydrofurane sec à 0°C. On ajoute 1,33 ml (10,5 mmoles) de complexe éther-trifluorure de bore, goutte à goutte, et on agite 30 min à 0°C (solution A). Parallèlement on place dans un tricol de 250 ml sous argon, 2,47 g (22 mmoles) de tertiobutylate de potassium sec dans 60 ml de tétrahydrofurane sec. On refroidit à -70°C et on coule, goutte à goutte, 22 ml d'une solution 1M de sec-butyl-lithium dans le mélange cyclohexane/hexane(22 mmoles)en maintenant la température inférieure à -60°C (solution B). Après la fin de l'addition,la solution A est cannulée dans la solution B en maintenant la température autour de -70°C. On laisse agiter 2 h.

Dans un tricol de 50 ml sous argon on place 2,36 ml (22 mmoles) de 4-fluorobenzaldéhyde distillé en solution dans 20 ml de tétrahydrofurane à -70°C. La solution B est cannulée en maintenant la température vers -70 °C. On laisse la solution résultante 30 min à -70°C et on laisse remonter à -20°C. On hydrolyse ensuite avec une solution d'acide chlorhydrique à 10%. On extrait à l'éther puis la phase aqueuse est reprise et basifiée avec de l'ammoniaque. On l'extrait au chloroforme puis on évapore le solvant sous pression réduite. Le résidu est purifié par flash chromatographie sur colonne de gel de silice en éluant avec un mélange de chloroforme et de méthanol. On obtient ainsi 0,53 g de 1-azabicyclo [2.2.2]oct-2-yl(4-fluorophényl)méthanol sous forme d'un solide jaunâtre
Point de fusion : 69-70°C.

5.2 1-azabicyclo[2.2.2]oct-2-yl(4-fluorophenyl)méthanone.

Dans un tricol de 250 ml sous azote on place 1,3 ml de diméthylsulfoxyde dans 40 ml de tétrahydrofurane à -70°C, on ajoute, goutte à goutte, 0,9 ml de chlorure d'oxalyle (11 mmoles) et on laisse sous agitation pendant 30 min à cette température. On ajoute une solution de 1 g (4,6 mmoles) de 1-azabicyclo[2.2.2]oct-2-yl(4-fluorophenyl)méthanol dans 40 ml de tétrahydrofurane, goutte à goutte. Après 30 min, 4 ml (27.6 mmoles) de triéthylamine sont ajoutés à -70°C. Le mélange réactionnel est alors agité 30 min à -70°C, 30 min à 0°C, puis 1 h à température ambiante.

Le mélange est versé dans une solution d'ammoniaque puis extrait plusieurs fois au chloroforme. Les phases organiques sont séchées sur sulfate de sodium et évaporées sous pression réduite.On purifie le résidu par chromatographie sur colonne de gel de silice en éluant avec un mélange de chloroforme et de méthanol. On obtient ainsi 1 g de 1-azabicyclo[2.2.2]oct-2-yl(4-fluorophényl)méthanone
Point de fusion : 68-69°C.

5.3 Chlorhydrate (Z) 1-azabicyclo[2.2.2]octyl(4-fluorophényl)méthanone *O*-benzyloxime.

Selon le mode opératoire décrit dans l'exemple 1.1, à partir de 1,17 g (5 mmoles) de cétone, on obtient 1,4 g de chlorhydrate de (Z) 1-azabicyclo[2.2.2]oct-yl(4-fluorophényl)méthanone *O*-benzyloxime après trituration dans l'éther du résidu obtenu après le traitement de la réaction.
Point de fusion : 202-203°C.

5.4 Thréo 1-azabicyclo[2.2.2]oct-yl(4-fluorophényl)-méthanamine.

Selon le mode opératoire décrit en 1.2, à partir de 1,47 g (4.54 mmoles) de chlorhydrate de (*Z*) 1-azabicyclo [2.2.2]octyl(4-fluorophényl)méthanone *O*-benzyloxime, on obtient 1 g de 1-azabicyclo[2.2.2]oct-yl(4-fluorophényl)méthanamine thréo (excès diastéréoisomérique, de=90%).

5.5 Chlorhydrate de *N*-[(*S*)-(2*S*)-1-azabicyclo[2.2.2]oct-2-yl(4-fluorophényl)méthyl]-2,6-dichloro-3-(trifluorométhyl) benzamide 1:1.

Selon le mode opératoire décrit en 1.3, à partir de 0,39 g (1,66 mmole) de thréo 1-azabicyclo[2.2.2]oct-yl(4-fluorophényl)méthanamine, 0,5g (1,83 mmole) de chlorure de l'acide 2,6-dichloro-3-trifluorométhylbenzoïque, 0,25 g (1,83 mmole) de carbonate de potassium, on obtient, après purification par chromatographie, 0,79 g de thréo *N*-[1-azabicyclo[2.2.2]oct-2-yl(4-fluorophényl)méthyl]-2,6-dichloro-3-(trifluorométhyl)benzamide sous forme d'une huile que l'on salifie avec une solution d'acide chlorhydrique gazeux dans l'éther éthylique.
Point de fusion : 290-291°C.

Les autres composés sont obtenus suivant les méthodes décrites dans les exemples 1, 2 et 5 à partir d'autres aldéhydes fonctionnalisés.

Le tableau 1 qui suit illustre les structures chimiques de quelques composés de l'invention.

Dans la colonne "R", $-CH{=}CH_2$ désigne un groupe vinyle , dans la colonne "$R_1$" $C_6H_5$ désigne un groupe phényle et 4-$C_9H_6N$ désigne un groupe quinoléin-4-yle. Dans la colonne "Sel", - désigne un composé à l'état de base, "HCl", désigne un chlorhydrate et "tfa", désigne un trifluoroacétate.

Les composés 14,19 à 23,24 du tableau se présentent sous la forme de chlorhydrate ou dichlorhydrate (voir tableau) solvaté par une ou plusieurs molécules d'eau .

Les composés 15 et 16 du tableau forment un couple d'énantiomères qui sont séparés par HPLC préparative en utilisant une colonne CHIRALCEL® AD 20μm et en tant que solvant un mélange isohexane/propan-2-ol 95/5 , de même pour les composés 17 et 18.

Le tableau 2 donne les propriétés physiques, points de fusion et pouvoirs rotatoires des composés du tableau. "(d)" indique un point de fusion avec décomposition.

Tableau 1

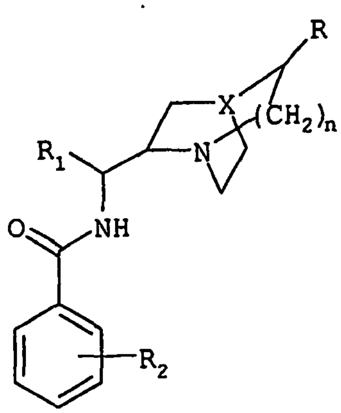

(I)

| N° | $R_1$ | R | X | n | $R_2$ | Sel | Stéréochimie |
|---|---|---|---|---|---|---|---|
| 1 | $C_6H_5$ | H | CH | 1 | 3-$SO_2N(CH_3)_2$, 4-C1 | - | thréo (1*R*, 2*R*; 1*S*, 2*S*) |
| 2 | $C_6H_5$ | H | CH | 1 | 2-C1, 5-$CF_3$ | HCl | thréo (1*R*, 2*R*; 1*S*, 2*S*) |
| 3 | $C_6H_5$ | H | CH | 1 | 2-C1, 3-$CF_3$ | HCl | thréo (1*R*, 2*R*; 1*S*, 2*S*) |
| 4 | $C_6H_5$ | H | CH | 1 | 2,6-$(Cl)_2$, 3-$CF_3$ | HCl | thréo (1*R*, 2*R*; 1*S*, 2*S*) |
| 5 | $C_6H_5$ | H | CH | 1 | 2-Cl, 3-$CH_3$, 6-F | HCl | thréo (1*R*, 2*R*; 1*S*, 2*S*) |
| 6 | $C_6H_5$ | H | CH | 1 | 2-Cl, 3-$CH_3$ | HCl | thréo (1*R*,2*R*;1*S*, 2*S*) |

(suite)

| N° | $R_1$ | R | X | n | $R_2$ | Sel | Stéréochimie |
|---|---|---|---|---|---|---|---|
| 7 | $C_6H_5$ | H | CH | 1 | 2,4,6-$(Cl)_3$ | HCl | thréo (1*R*,2*R*;1*S*, 2*S*) |
| 8 | $C_6H_5$ | H | CH | 1 | 2-$CH_3$, 3-$CF_3$ | HCl | thréo (1*R*,2*R*;1*S*, 2*S*) |
| 9 | $C_6H_5$ | H | CH | 1 | 2,6-$(Cl)_2$ | HCl | thréo (1*R*,2*R*;1*S*, 2*S*) |
| 10 | $C_6H_5$ | H | CH | 1 | 2,5-$(CF_3)_2$ | HCl | thréo (1*R*,2*R*;1*S*, 2*S*) |
| 11 | $C_6H_5$ | H | CH | 1 | 2-F, 3-Cl, 6-$CF_3$ | HCl | thréo (1*R*,2*R*;1*S*, 2*S*) |
| 12 | $C_6H_5$ | H | CH | 1 | 2-$CH_3$, 3-Cl | HCl | thréo (1*R*,2*R*;1*S*, 2*S*) |
| 13 | $C_6H_5$ | H | CH | 1 | 2,3-$(Cl)_2$ | HCl | thréo (1*R*,2*R*;1*S*, 2*S*) |
| 14 | 4-$C_9H_6N$ | $CH=CH_2$ | CH | 1 | 2-Cl, 3-$CF_3$ | 2HCl | (1*S*,2*S*) |
| 15 | $C_6H_5$ | H | CH | 1 | 2-Cl, 3-$CF_3$ | tfa | (1*R*,2*R*) |
| 16 | $C_6H_5$ | H | CH | 1 | 2-Cl, 3-$CF_3$ | tfa | (1*S*,2*S*) |
| 17 | $C_6H_5$ | H | CH | 1 | 2, 6- $(Cl)_2$, 3-$CF_3$ | HCl | (1*S*,2*S*) |
| 18 | $C_6H_5$ | H | CH | 1 | 2, 6- $(Cl)_2$, 3-$CF_3$ | HCl | (1*R*,2*R*) |
| 19 | $C_6H_5$ | H | N | 1 | 2-Cl, 3-$CF_3$ | 2HCl | thréo (1*R*, 2*R*; 1*S*, 2*S*) |
| 20 | $C_6H_5$ | H | N | 1 | 2, 6- $(Cl)_2$, 3-$CF_3$ | 2HCl | thréo (1*R*, 2*R*; 1*S*, 2*S*) |
| 21 | $C_6H_5$ | H | N | 1 | 2,6-$(Cl)_2$ | 2HCl | thréo (1*R*, 2*R*; 1*S*, 2*S*) |
| 22 | $C_6H_5$ | H | N | 1 | 2-$CH_3$, 3-$CF_3$ | 2HCl | thréo (1*R*, 2*R*; 1*S*, 2*S*) |
| 23 | $C_6H_5$ | H | N | 1 | 2-$CH_3$, 3-Cl | 2HCl | thréo (1*R*, 2*R*; 1*S*, 2*S*) |
| 24 | $C_6H_5$ | H | CH | 1 | 3, 5 $(Cl)_2$, 4-$NH_2$ | HCl | thréo (1*R*, 2*R*; 1*S*, 2*S*) |
| 25 | 4-F-$C_6H_4$ | H | CH | 1 | 2-Cl,3-$CF_3$ | HCl | thréo (1*R*,2*R*;1*S*, 2*S*) |
| 26 | 4-F-$C_6H_4$ | H | CH | 1 | 2,6-$(Cl)_2$, 3-$CF_3$ | HCl | thréo (1*R*,2*R*;1*S*, 2*S*) |
| 27 | 1-naphtyle | H | CH | 1 | 2,6-$(Cl)_2$,3-$CF_3$ | HCl | thréo (1*R*,2*R*;1*S*, 2*S*) |
| 28 | 1-naphtyle | H | CH | 1 | 2-Cl,3-$CF_3$ | HCl | thréo (1*R*,2*R*;1*S*, 2*S*) |
| 29 | 1-naphtyle | H | CH | 1 | 2-$CH_3$,3-Cl | HCl | thréo (1*R*,2*R*;1*S*, 2*S*) |
| 30 | 2-$CH_3$ -$C_6H_4$ | H | CH | 1 | 2-Cl,3-$CF_3$ | HCl | thréo (1*R*,2*R*;1*S*, 2*S*) |

(suite)

| N° | $R_1$ | R | X | n | $R_2$ | Sel | Stéréochimie |
|---|---|---|---|---|---|---|---|
| 31 | 2-$CH_3$ -$C_6H_4$ | H | CH | 1 | 2,6-$(Cl)_2$, 3-$CF_3$ | HCl | thréo (1*R*,2*R*;1*S*, 2*S*) |
| 32 | $C_6H_5$ | H | CH | 1 | 6-$CH_3$, 3-Cl, 2-$NH_2$ | HCl | thréo (1*R*,2*R*;1*S*, 2*S*) |
| 33 | $C_6H_5$ | H | CH | 1 | 3, 6- (Cl) $_2$, 2-$NH_2$ | HCl | thréo (1*R*,2*R*;1*S*, 2*S*) |
| 34 | $C_6H_5$ | H | CH | 1 | 2,6-$(Cl)_2$, 3-$CH_3$ | HCl | thréo (1*R*,2*R*;1*S*, 2*S*) |
| 35 | $C_6H_5$ | H | CH | 1 | 2-$CH_3$, 3-Cl | HCl | (1*S*, 2*S*) |
| 36 | $C_6H_5$ | H | CH | 1 | 2-Cl, 3-$CF_3$ | HCl | érythro (1*S*, 2*R;* 1*R*, 2*S*) |
| 37 | 4-F-$C_6H_4$ | H | CH | 1 | 2-$CH_3$, 3-Cl | HCl | thréo/érythro: 9/1 |
| 38 | 3-F-$C_6H_4$ | H | CH | 1 | 2, 6- $(Cl)_2$, 3-$CF_3$ | HCl | thréo (1*R*, 2*R;* 1*S*, 2*S*) |
| 39 | 3-F-$C_6H_4$ | H | CH | 1 | 2,6-$(Cl)_2$, 3-$CF_3$ | HCl | érythro (1*S*, 2*R;* 1*R*, 2*S*) |
| 40 | 4-F, 3-$CH_3$-$C_6H_3$ | H | CH | 1 | 2-$CH_3$, 3-$OCH_3$ | HCl | thréo/érythro 1/1 |
| 41 | 9-F,3-$CH_3$-$C_6H_3$ | H | CH | 1 | 3,5-$(OCH_3)_2$ | HCl | thréo/érythro 1/1 |
| 42 | 4-F,3-$CH_3$-$C_6H_3$ | H | CH | 1 | 2,6-$(Cl)_2$,3-$CF_3$ | HCl | thréo (1*R*,2*R*;1*S*, 2*S*) |
| 43 | 3-F-$C_6H_4$ | H | CH | 1 | 2-$CH_3$,3-$OCH_3$ | HCl | thréo (1*R*,2*R*; 1*S*, 2*S*) |
| 44 | $C_6H_5$ | H | CH | 1 | 2-$OCH_3$,5-Cl | HCl | (1*S*,2*S*) |
| 45 | $C_6H_5$ | H | CH | 1 | 2-Br,5-$OCH_3$ | HCl | (1*S*,2*S*) |
| 46 | $C_6H_5$ | H | CH | 1 | 2,3-($OCF_2O$) | HCl | (1*S*,2*S*) |
| 47 | $C_6H_5$ | H | CH | 1 | 2,6-$(OCH_3)$ $_2$ | HCl | (1*S*,2*S*) |
| 48 | $C_6H_5$ | H | CH | 1 | 3,5-$(OCH_3)$ $_2$ | HCl | (1*S*,2*S*) |
| 49 | $C_6H_5$ | H | CH | 1 | 2,3-$(OCH_3)$ $_2$ | HCl | (1*S*,2*S*) |
| 50 | $C_6H_5$ | H | CH | 1 | 2-$NH_2$,3-$CH_3$ | 2HCl | (1*S*,2*S*) |
| 51 | $C_6H_5$ | H | CH | 1 | 2-$OCH_3$, 3, 6- (Cl) $_2$ | HCl | (1*S*,2*S*) |
| 52 | $C_6H_5$ | H | CH | 1 | 3-(O-$C_6H_5$) | HCl | (1*S*,2*S*) |
| 53 | $C_6H_5$ | H | CH | 1 | 2,5-$(OCH_3)_2$ | HCl | (1*S*,2*S*) |
| 54 | $C_6H_5$ | H | CH | 1 | 2-$CH_3$,3-$OCH_3$ | HCl | (1*S*,2*S*) |
| 55 | $C_6H_5$ | H | CH | 1 | 2-$OCH_3$, 3, 5 $(Cl)_2$ | HCl | (1*S*,2*S*) |
| 56 | $C_6H_5$ | H | CH | 1 | 2-Cl, 6-$CH_3$ | HCl | (1*S*,2*S*) |
| 57 | $C_6H_5$ | H | CH | 1 | 2-$NH_2$, 6-$CH_3$ | 2HCl | (1*S*, 2*S*) |
| 58 | $C_6H_5$ | H | CH | 1 | 2-$NH_2$, 5-Br | 2HCl | (1*S*, 2*S*) |
| 59 | $C_6H_5$ | H | CH | 1 | 2-$NH_2$, 5-$CH_3$ | 2HCl | (1*S*, 2*S*) |
| 60 | $C_6H_5$ | H | CH | 1 | 2-$OCH_3$, 3-$CH_3$ | HCl | (1*S*, 2*S*) |
| 61 | $C_6H_5$ | H | CH | 1 | 2-$NH_2$, 5-$OCH_3$ | 2HCl | (1*S*, 2*S*) |

(suite)

| N° | $R_1$ | R | X | n | $R_2$ | Sel | Stéréochimie |
|---|---|---|---|---|---|---|---|
| 62 | $C_6H_5$ | H | CH | 1 | 2-$NH_2$, 3-$OCH_3$ | 2HCl | (1*S*, 2*S*) |
| 63 | $C_6H_5$ | H | CH | 1 | 2-C1, 5-$CH_3$ | HCl | (1*S*, 2*S*) |
| 64 | $C_6H_5$ | H | CH | 1 | 2-$OCH_3$, 5-$CH_3$ | HCl | (1*S*, 2*S*) |
| 65 | $C_6H_5$ | H | CH | 1 | 2-$CH_3$, 3-OH | HCl | (1*S*, 2*S*) |
| 66 | $C_6H_5$ | H | CH | 1 | 2-$CH_3$, 5-Cl | HCl | (1*S*, 2*S*) |
| 67 | 4-$OCH_3$-$C_6H_4$ | H | CH | 1 | 2,6-$(Cl)_2$, 3-$CF_3$ | HCl | thréo (1*R*,2*R*;1*S*, 2*S*) |
| 68 | 4-OH-$C_6H_4$ | H | CH | 1 | 2, 6- $(Cl)_2$, 3-$CF_3$ | - | thréo (1*R*,2*R*;1*S*, 2*S*) |
| 69 | 3-F-$C_6H_4$ | H | CH | 1 | 2-$CH_3$, 3-Cl | HCl | thréo (1*R*,2*R*;1*S*, 2*S*) |

Tableau 2

| N° | F (°C) | $[\alpha]_D^{25}$ (°) |
|---|---|---|
| 1 | 233-235 | - |
| 2 | 267-269 | - |
| 3 | 257-262 | - |
| 4 | 270-273 | - |
| 5 | 315-316 | - |
| 6 | 319-320 | - |
| 7 | >300 | - |
| 8 | 281-283 | - |
| 9 | 359-361 | - |
| 10 | 281-283 | - |
| 11 | 347-349 | - |
| 12 | 311-313 | - |
| 13 | 316-318 | - |
| 14 | 185-205 | -5,4 (c=0,986 MeOH) |
| 15 | 196-197 | -51.3 (c=1,03 MeOH) |
| 16 | 214-215 | +48.2 (c=0,618 MeOH) |
| 17 | 264-268°C | +61.1 (c=0,32 MeOH) |
| 18 | 265-268 | -58.9 (c=0,3 MeOH) |
| 19 | 207-208 | - |
| 20 | 214-215 | - |
| 21 | 210-211 | - |
| 22 | 215-217 | - |
| 23 | 210-212 | - |
| 24 | 336-339 | - |
| 25 | 271-273 | - |
| 26 | 290-291 | - |
| 27 | 317-318 | - |
| 28 | 314-315 | - |
| 29 | 315-316 | - |
| 30 | 215-230 | - |
| 31 | 210-220 | - |

(suite)

| N° | F (°C) | $[\alpha]_D^{25}$ (°) |
|---|---|---|
| 32 | 328-330 | - |
| 33 | 275-280 | - |
| 34 | 338-344 | - |
| 35 | 295-300 | +55,2 (c=0,3 MeOH) |
| 36 | 287-291 | - |
| 37 | >300 | - |
| 38 | 232-234 | - |
| 39 | 289-291 | - |
| 40 | 124-126 | - |
| 41 | 154-156 | - |
| 42 | 254-256 | - |
| 43 | 280-282 | - |
| 44 | 162-164 | +87,4 (c=0,32 ; MeOH) |
| 45 | 275-277 | +43,8 (c=0,32 ; MeOH) |
| 46 | 191-193 | +42,4 (c=0,32 ; MeOH) |
| 47 | 234-236 | +53,2 (c=0,30 ; MeOH) |
| 48 | 297-299 | +21,3 (c=0,31 ; MeOH) |
| 49 | 284-286 | +68,6 (c=0,32 ; MeOH) |
| 50 | 244-246 | +41,1 (c=0,30 ; MeOH) |
| 51 | 194-196 | +73,9 (c=0,29 ; MeOH) |
| 52 | 105-108 | +26,7 (c=0,30 ; MeOH) |
| 53 | 169-171 | +82,3 (c=0,30 ; MeOH) |
| 54 | 298-300 | +46,6 (c=0,31 ; MeOH) |
| 55 | 213-215 | +75,9 (c=0,30 ; MeOH) |
| 56 | 331-333 | +67,9 (c=0,31 ; MeOH) |
| 57 | 295-297 | +79,4 (c=0,30 ; MeOH) |
| 58 | 244-246 | +16,1 (c=0,30 ; MeOH) |
| 59 | 282-284 | +26,4 (c=0,31 ; MeOH) |
| 60 | 235-237 | +116,6 (c=0,29 ; MeOH) |
| 61 | 278-280 | +14,2 (c=0,30 ; MeOH) |
| 62 | 264-266 | +40,5 (c=0,30 ; MeOH) |
| 63 | 128-130 | +61,9 (c=0,32 ; MeOH) |
| 64 | 185-187 | +81,9 (c=0,30 ; MeOH) |
| 65 | 329-331 | +45,9 (c=0,29 ; MeOH) |
| 66 | 242-244 | +8,4 (c=0,31; MeOH) |
| 67 | 284-286 | - |
| 68 | | - |
| 69 | 291-293 | - |

Les composés de l'invention ont été soumis à une série d'essais pharmacologiques qui ont mis en évidence leur intérêt comme substances à activités thérapeutiques.

<u>Etude du transport de la glycine dans les cellules SK-N-MC exprimant le transporteur humain glyt1 natif.</u>

La capture de [$^{14}$C]glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-1-éthanesulfonique) à pH 7,4. Après 10 min de préincubation à 37°C en présence soit de tampon (lot témoin), soit de composé à tester a différentes concentrations ou de 10 mM de glycine (détermination

de la capture non spécifique), 10 µM de [$^{14}$C]glycine (activité spécifique 112 mCi/mmole) sont ensuite ajoutés. L'incubation se poursuit pendant 10 min à 37°C, et la réaction est arrêtée par 2 lavages avec un tampon Krebs-HEPES à pH 7,4. La radioactivité incorporée par les cellules est alors estimée après ajout de 100 µl de scintillant liquide et agitation pendant 1 h. Le comptage est réalisé sur compteur Microbeta Tri-lux™. L'efficacité du composé est déterminée par la $CI_{50}$, concentration du composé qui diminue de 50% la capture spécifique de glycine, définie par la différence de radioactivité incorporée par le lot témoin et le lot qui a reçu la glycine à 10 mM.

Les composés les plus actifs de l'invention, dans ce test, ont une $CI_{50}$ de l'ordre de 0,001 à 10 µM.

Les résultats individuels de quelques composés sont les suivants ($CI_{50}$ en µM) :

| | |
|---|---|
| Composé N°3 | 0,017 |
| Composé N°4 | 0,004 |
| Composé N°14 | 0,07 |
| Composé N°17 | 0,001 |
| Composé N°26 | 0,07 |

Etude *ex vivo* de l'activité inhibitrice d'un composé sur la capture de la [$^{14}$C]glycine dans l'homogénat cortical de souris.

Des doses croissantes du composé à étudier sont administrées par voie orale (préparation par trituration de la molécule à tester dans un mortier dans une solution de Tween/Methocel™ à 0,5% dans de l'eau distillée) ou intrapéritonéale (dissolution de la molécule à tester dans du sérum physiologique ou prépation par trituration dans un mortier dans une solution de Tween/Methocel™ à 0,5% dans de l'eau, selon la solubilité de la molécule) sur des souris mâles OF1 Iffa Crédo de 20 à 25 g le jour de l'expérience. Le groupe témoin est traité par le véhicule. Les doses en mg/kg, la voie d'administration et le temps de traitement sont déterminés en fonction de la molécule à étudier.

Après euthanasie par décapitation des animaux à un temps donné après l'administration, le cortex de chaque animal est rapidement prélevé sur glace, pesé et conservé à 4°C ou congelé à -80°C (dans les deux cas les échantillons sont conservés 1 jour maximum). Chaque échantillon est homogénéisé dans un tampon Krebs-HEPES à pH 7,4 à raison de 10 ml/g de tissu. 20 µl de chaque homogénat sont incubés pendant 10 min à température ambiante en présence de 10 mM de L-alanine et de tampon. La capture non spécifique est déterminée par addition de 10 mM de glycine au groupe témoin. La réaction est arrêtée par filtration sous vide et la radioactivité retenue est estimée par scintillation solide par comptage sur compteur Microbeta Tri-lux™.

Un inhibiteur de la capture de la [$^{14}$C]glycine diminuera la quantité de radioligand incorporée dans chaque homogénat. L'activité du composé est évaluée par sa $DE_{50}$, dose qui inhibe 50% de la capture de la [$^{14}$C]glycine par rapport au groupe témoin.

Les composés de l'invention les plus puissants, dans ce test, ont une $DE_{50}$ de 0,1 à 5 mg/kg par voie intrapéritonéale ou par voie orale.

Etude du transport de la glycine dans l'homogénat de moelle épinière de souris.

La capture de [$^{14}$C]glycine par le transporteur glyt2 est étudiée dans l'homogénat de moelle épinière de souris par la mesure de radioactivité incorporée en présence ou en absence de composé à étudier.

Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu.

50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [$^{14}$C]glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux™.

L'efficacité du composé est déterminée par la concentration $CI_{50}$ capable de diminuer de 50% la capture spécifique de glycine, définie par la différence de radioactivité incorporée par le lot témoin et le lot qui a reçu la glycine 10 mM.

Les composés les plus actifs de l'invention dans ce test, ont une $CI_{50}$ de l'ordre de 0,02 à 10 µM. La $CI_{50}$ du composé N°17 est de 0,69µM.

Les résultats des essais effectués sur les composés de l'invention de formule générale (I) montrent qu'ils sont des inhibiteurs des transporteurs de la glycine glyt1, présents majoritairement dans le cerveau, et des transporteurs de la glycine glyt2, présents majoritairement dans la moelle épinière.

Les composés selon l'invention peuvent donc être utilisés pour la préparation de médicaments, en particulier de médicaments inhibiteurs des transporteurs de la glycine glyt1 et/ou glyt2.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Les composés de l'invention peuvent être utilisés notamment pour le traitement des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Par ailleurs, les composés de l'invention peuvent être utilisés pour le traitement des contractures musculaires douloureuses en rhumatologie et en pathologie rachidienne aiguë, pour le traitement des contractures spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

La présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec un ou plusieurs excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques (« patch »), des suppositoires. Pour l'administration topique on peut envisager des pommades, lotions et collyres.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule générale (I)

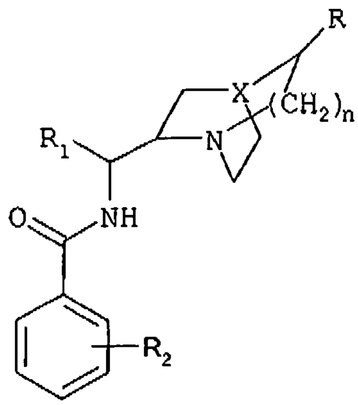

(I)

dans laquelle

R représente un atome d'hydrogène ou un groupe vinyle;
n représente 0 ou 1 ou 2 quand R représente un atome d'hydrogène et n représente 1 quand R représente un groupe vinyle ;
X représente un groupe de formule CH ou un atome d'azote quand R représente un atome d'hydrogène et X représente un groupe de formule CH quand R représente un groupe vinyle ;
$R_1$ représente soit un groupe phényle ou naphtyle éventuellement substitué par un ou plusieurs substituants choisis parmi les atomes d'halogène, les groupes $(C_1-C_6)$alkyle, hydroxy et $(C_1-C_6)$alcoxy linéaires ou ramifiés, le groupe trifluorométhyle, soit un groupe cyclohexyle, soit un groupe hétéroaryle choisi parmi les groupes thiényle, pyridinyle, oxazolyle, furanyle, thiazolyle, quinoléinyle et isoquinoléinyle ;
$R_2$ représente soit un atome d'hydrogène, soit un ou plusieurs substituants choisis parmi les atomes d'halogène et les groupes trifluorométhyle, $(C_1-C_6)$alkyle, $(C_1-C_6)$alcoxy, thiényle, phényloxy, hydroxy, mercapto, thio $(C_1-C_6)$alkyle, cyano ou un groupe de formule générale $-NR_4R_5$, $SO_2NR_4R_5$, $-SO_2-(C_1-C_6)$alkyle, $-SO_2$-phényle, $-CONR_4R_5$, $-COOR_7$, $-CO-(C_1-C_6)$alkyle, -CO-phényle, - $NHCOR_8$, $-NHSO_2-(C_1-C_6)$ alkyle, $-NHSO_2$-phényle et $-NHSO_2NR_4R_5$ ou un groupe de formule $-OCF_2O-$ lié aux positions 2 et 3 du groupe phényle ;
les groupes $(C_1-C_6)$alkyle, $(C_1-C_6)$alcoxy, $-SO_2-(C_1-C_6)$alkyle, $-CO-(C_1-C_6)$alkyle et $-NHSO_2-(C_1-C_6)$alkyle étant éventuellement substitués par un ou plusieurs groupes $R_3$ ;
les groupes phényle, $-SO_2$-phényle, -CO-phényle et - $NHSO_2$-phényle étant éventuellement substitués par un groupe $R_6$ ; $R_3$ représente un atome d'halogène, un groupe phényle, $(C_1-C_6)$alcoxy, $-NR_4R_5$ ;
$R_4$ et $R_5$ représentent indépendamment l'un de l'autre un atome d'hydrogène ou un groupe $(C_1-C_6)$alkyle ou $R_4$ et $R_5$ forment avec l'atome d'azote qui les porte un cycle pyrrolidine, un cycle pipéridine ou un cycle morpholine ;
$R_6$ représente un atome d'hydrogène, un atome d'halogène, un groupe trifluorométhyle,un groupe cyano, un groupe hydroxy, un groupe mercapto, un groupe $(C_1-C_6)$alkyle ou $(C_1-C_6)$alcoxy ; $R_7$ représente un atome d'hydrogène ou un groupe $(C_1-C_6)$alkyle éventuellement substitué par un ou plusieurs groupes $R_3$, ou un groupe phényle éventuellement substitué par un groupe $R_6$;
$R_8$ représente un groupe $(C_1-C_6)$alkyle éventuellement substitué par un ou plusieurs groupes $R_3$, ou un groupe $(C_1-C_6)$alcoxy, ou un groupe phényle éventuellement substitué par un groupe $R_6$,
à l'état de base libre ou de sel d'addition à un acide, d'hydrate ou de solvat.

2. Composé selon la revendication 1, **caractérisé en ce qu'**il est de configuration thréo.

3. Composé selon l'une des revendications 1 et 2, **caractérisé en ce que** n représente 0 ou 1.

4. Composé selon la revendication 3, **caractérisé en ce que** X représente un groupe de formule CH.

5. Composé selon la revendication 4, **caractérisé en ce que** R représente un atome d'hydrogène.

6. Composé selon la revendication 5, **caractérisé en ce que** n représente 1.

7. Composé selon la revendication 6, **caractérisé en ce que** $R_1$ représente un groupe phényle éventuellement substitué.

**8.** Composé selon la revendication 1, **caractérisé en ce qu'**il est choisi parmi les composés suivants :

thréo-2-chloro-*N*-[(1-azabicyclo[2.2.2]oct-2-yl)phényl méthyl]-3-trifluorométhylbenzamide ;
Chlorhydrate de thréo-2-chloro-*N*-[(1-azabicyclo[2.2.2]oct-2-yl)phénylméthyl]-3-trifluorométhylbenzamide ;
thréo-2,6-dichloro-*N*-[(1-azabicyclo[2.2.2]oct-2-yl)phényl méthyl]-3-trifluorométhylbenzamide ;
Chlorhydrate de thréo-2,6-dichloro-*N*-[(1-azabicyclo[2.2.2]oct-2-yl)phénylméthyl]-3-trifluorométhylbenzamide ;
2-chloro-N-(8α,9*S*-cinchonan-9-yl)-3-trifluorométhylbenzamide ; Chlorhydrate de 2-chloro-N-(8α,9*S*-cinchonan-9-yl)-3-trifluorométhylbenzamide ;
2,6-dichloro-*N*-[(1*S*)-[(2*S*) (1-azabicyclo[2.2.2]oct-2-yl)phényl méthyl]-3-(trifluoxométhyl)benzamide :
Chlorhydrate de 2,6-dichloro-*N*-[(1*S*)-[(2*S*) (1-azabicyclo[2.2.2]oct-2-yl)phénylméthyl]-3-(trifluorométhyl) benzamide ;
thréo *N*-[1-azabicyclo[2.2.2]oct-2-yl(4-fluorophenyl)methyl]-2,6-dichloro-3-(trifluoromethyl)benzamide ;
Chlorhydrate de thréo *N*-[1-azabicyclo[2.2.2]oct-2-yl(4-fluorophenyl)methyl]-2,6-dichloro-3-(trifluoromethyl) benzamide.

**9.** Médicament **caractérisé en ce qu'**il consiste en un composé selon l'une des revendications 1 à 8.

**10.** Composition pharmaceutique, **caractérisée en ce qu'**elle comprend un composé selon l'une des revendications 1 à 8, ainsi qu'au moins un excipient pharmaceutiquement acceptable.

**11.** Procédé de préparation d'un composé selon la revendication 1, **caractérisé en ce que** l'on fait réagir une diamine de formule générale (II)

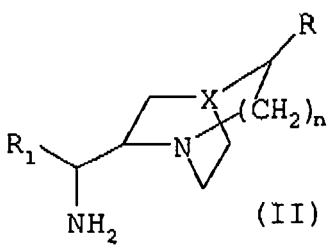

(II)

dans laquelle n, R, et $R_1$ sont tels que définis selon la revendication 1 avec un acide activé ou un composé de formule générale (III)

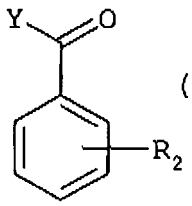

(III)

dans laquelle Y représente un groupe nucléofuge.

**12.** Procédé de préparation d'un composé selon la revendication 1, **caractérisé en ce que** le groupe nucléofuge est un halogène.

**13.** Procédé de préparation d'un composé selon la revendication 11, **caractérisé en ce qu'**il comprend un composé choisi parmi les composés suivants :

- Chlorhydrate de (*Z*)-1-azabicyclo[2.2.2]oct-2-yl(phényl)méthanone *O*-benzyloxime ;
- thréo[1-azabicyclo[2.2.2]oct-2-yl(phényl)méthyl]amine ;
- chlorhydrate de thréo-2-chloro-N-[(1-azabicyclo[2.2.2]oct-2-yl)phényl]méthyl]amine ;
- 8α,9*S*-cinchonan-9-amine ;
- Chlorhydrate de 2-chloro-*N*-(8α,9*S*-cinchonan-9-yl)-3-trifluorométhylbenzamide ;

- D-tartrate de (1*S*)-[(2*S*)-1-azabicyclo[2.2.2.]oct-2-yl(phényl)méthyl]amine ;
- 1-azabicylo[2.2.2]oct-2-yl(4-fluorophényl)méthanol;
- 1-azabicylo[2.2.2]oct-2-yl(4-fluorophenyl)méthanone ;
- Chlorhydrate (*Z*) 1-azabicyclo[2.2.2]octyl(4-fluorophényl)méthanone *O*-benzyloxime ;
- Thréo 1-azabicyclo[2.2.2]oct-yl(4-fluorophényl)méthanamine.

**14.** Utilisation d'un composé de formule (I) selon l'une des revendications 1 à 8, pour la préparation d'un médicament destiné au traitement des troubles comportementaux associés à la démence, des psychoses, des diverses formes d'anxiété, des attaques de panique, des phobies, des troubles obsessionnels compulsifs, des différentes formes de dépression, des troubles dus à l'abus ou au sevrage d'alcool, des troubles du comportement sexuel, des troubles de la prise de nourriture et de la migraine.

**15.** Utilisation d'un composé de formule (I) selon l'une des revendications 1 à 8, pour la préparation d'un médicament destiné au traitement des contractures, de la douleur, de la maladie de Parkinson et des symptômes parkinsoniens, des épilepsies, des formes mixtes et autres syndromes épileptiques en complément d'un autre traitement antiépileptique, ou en monothérapie, des apnées du sommeil, et pour la neuroprotection.

## Claims

**1.** Compound corresponding to general formula (I)

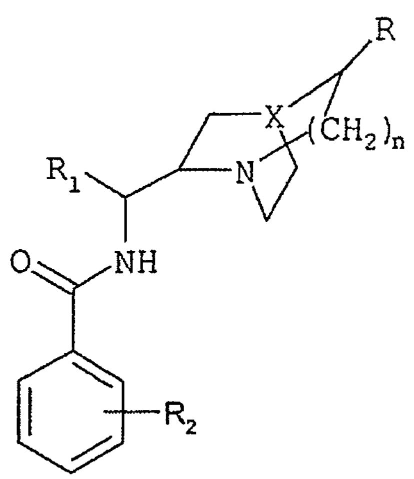

(I)

in which

R represents a hydrogen atom or a vinyl group;
n represents 0 or 1 or 2 when R represents a hydrogen atom and n represents 1 when R represents a vinyl group;
X represents a group of formula CH or a nitrogen atom when R represents a hydrogen atom and X represents a group of formula CH when R represents a vinyl group;
$R_1$ represents either a phenyl or naphthyl group optionally substituted with one or more substituents chosen from halogen atoms, linear or branched $(C_1\text{-}C_6)$alkyl, hydroxyl and $(C_1\text{-}C_6)$alkoxy groups, the trifluoromethyl group, or a cyclohexyl group, or a heteroaryl group chosen from the thienyl, pyridinyl, oxazolyl, furanyl, thiazolyl, quinolinyl, and isoquinolinyl groups;
$R_2$ represents either a hydrogen atom, or one or more substituents chosen from halogen atoms and the trifluoromethyl, $(C_1\text{-}C_6)$alkyl, $(C_1\text{-}C_6)$alkoxy, thienyl, phenyloxy, hydroxyl, mercapto, thio$(C_1\text{-}C_6)$alkyl and cyano groups or a group of general formula $-NR_4R_5$, $SO_2NR_4R_5$, $-SO_2\text{-}(C_1\text{-}C_6)$alkyl, $-SO_2$-phenyl, $-CONR_4R_5$, $-COOR_7$, $-CO\text{-}(C_1\text{-}C_6)$ alkyl, -CO-phenyl, $-NHCOR_8$, $-NHSO_2\text{-}(C_1\text{-}C_6)$-alkyl, $-NHSO_2$-phenyl and $-NHSO_2NR_4R_5$ or a group of formula $-OCF_2O-$ attached at the 2- and 3-positions of the phenyl group;
the groups $(C_1\text{-}C_6)$alkyl, $(C_1\text{-}C_6)$alkoxy, $-SO_2\text{-}(C_1\text{-}C_6)$alkyl, $-CO\text{-}(C_1\text{-}C_6)$alkyl and $-NHSO_2\text{-}(C_1\text{-}C_6)$alkyl being optionally substituted with one or more groups $R_3$;
the groups phenyl, $-SO_2$-phenyl, -CO-phenyl and $-NHSO_2$-phenyl being optionally substituted with a group $R_6$;

$R_3$ represents a halogen atom, or a phenyl, $(C_1-C_6)$alkoxy or $-NR_4R_5$ group;
$R_4$ and $R_5$ represent, independently of each other, a hydrogen atom or a $(C_1-C_6)$alkyl group or $R_4$ and $R_5$ form with the nitrogen atom bearing them a pyrrolidine ring, a piperidine ring or a morpholine ring;
$R_6$ represents a hydrogen atom, a halogen atom, a trifluoromethyl group, a cyano group, a hydroxyl group, a mercapto group, a $(C_1-C_6)$alkyl or $(C_1-C_6)$alkoxy group;
$R_7$ represents a hydrogen atom or a $(C_1-C_6)$alkyl group optionally substituted with one or more groups $R_3$, or a phenyl group optionally substituted with a group $R_6$;
$R_8$ represents a $(C_1-C_6)$alkyl group optionally substituted with one or more groups $R_3$, or a $(C_1-C_6)$alkoxy group, or a phenyl group optionally substituted with a group $R_6$,

in the form of a free base or of an addition salt with an acid, of a hydrate or of a solvate.

2. Compound according to Claim 1, **characterized in that** it is of the threo configuration.

3. Compound according to either of Claims 1 and 2, **characterized in that** n represents 0 or 1.

4. Compound according to Claim 3, **characterized in that** X represents a group of formula CH.

5. Compound according to Claim 4, **characterized in that** R represents a hydrogen atom.

6. Compound according to Claim 5, **characterized in that** n represents 1.

7. Compound according to Claim 6, **characterized in that** $R_1$ represents an optionally substituted phenyl group.

8. Compound according to Claim 1, **characterized in that** it is chosen from the following compounds:

threo-2-chloro-*N*-[(1-azabicyclo[2.2.2]oct-2-yl)-phenylmethyl]-3-trifluoromethylbenzamide;
threo-2-chloro-*N*-[(1-azabicyclo[2.2.2]oct-2-yl)-phenylmethyl]-3-trifluoromethylbenzamide hydrochloride;
threo-2,6-dichloro-*N*-[(1-azabicyclo[2.2.2]oct-2-yl)-phenylmethyl]-3-trifluoromethylbenzamide;
threo-2,6-dichloro-*N*-[(1-azabicyclo[2.2.2]oct-2-yl)-phenylmethyl]-3-trifluoromethylbenzamide hydrochloride;
2-chloro-N-(8α,9*S*-cinchonan-9-yl)-3-trifluoromethyl-benzamide;
2-chloro-N-(8α,9*S*-cinchonan-9-yl)-3-trifluoromethyl-benzamide hydrochloride;
2,6-dichloro-*N*-[(1*S*)-[(2*S*) (1-azabicyclo[2.2.2]oct-2-yl)phenylmethyl]-3-(trifluoromethyl)benzamide;
2,6-dichloro-N-[(1*S*)-[(2*S*) (1-azabicyclo[2.2.2]oct-2-yl)phenylmethyl]-3-(trifluoromethyl)benzamide hydrochloride;
threo-*N*-[1-azabicyclo[2.2.2]oct-2-yl(4-fluorophenyl)-methyl]-2,6-dichloro-3-(trifluoromethyl)benzamide;
threo-*N*-[1-azabicyclo[2.2.2]oct-2-yl(4-fluorophenyl)-methyl]-2,6-dichloro-3-(trifluoromethyl)benzamide hydrochloride.

9. Medicament, **characterized in that** it consists of a compound according to one of Claims 1 to 8.

10. Pharmaceutical composition, **characterized in that** it comprises a compound according to one of Claims 1 to 8, and at least one pharmaceutically acceptable excipient.

11. Process for preparing a compound according to Claim 1, **characterized in that** a diamine of general formula (II)

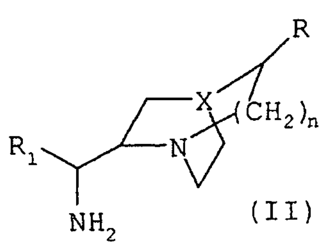

(II)

in which n, R and $R_1$ are as defined according to Claim 1, is reacted with an activated acid or a compound of general

formula (III)

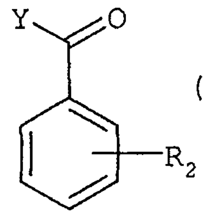

(III)

in which Y represents a leaving group.

**12.** Process for preparing a compound according to Claim 1, **characterized in that** the leaving group is a halogen.

**13.** Process for preparing a compound according to Claim 11, **characterized in that** it comprises a compound chosen from the following compounds:

- (*Z*)-1-azabicyclo[2.2.2]oct-2-yl(phenyl)methanone *O*-benzyloxime hydrochloride;
- threo[1-azabicyclo[2.2.2]oct-2-yl(phenyl)methyl]-amine;
- threo-2-chloro-N-[(1-azabicyclo[2.2.2]oct-2-yl)-phenylmethyl]amine hydrochloride;
- 8α,9*S*-cinchonan-9-amine;
- 2-chloro-*N*-(8α,9*S*-cinchonan-9-yl)-3-trifluoromethyl-benzamide hydrochloride;
- (1*S*)-[(2*S*)-1-azabicyclo[2.2.2]oct-2-yl(phenyl)-methyl]amine D-tartrate;
- 1-azabicyclo[2.2.2]oct-2-yl(4-fluorophenyl)methanol;
- 1-azabicyclo[2.2.2]oct-2-yl(4-fluorophenyl)methanone;
- (*Z*)-1-azabicyclo[2.2.2]octyl(4-fluorophenyl)methanone *O*-benzyloxime hydrochloride;
- threo-1-azabicyclo[2.2.2]oct-yl(4-fluorophenyl)-methanamine.

**14.** Use of a compound of formula (I) according to one of Claims 1 to 8 for the preparation of a medicament intended for the treatment of behavioural disorders associated with dementia, of psychoses, of various forms of anxiety, panic attacks, phobias, obsessive-compulsive disorders, of various forms of depression, of disorders due to alcohol abuse or to withdrawal from alcohol, sexual behaviour disorders, food intake disorders, and of migraine.

**15.** Use of a compound of formula (I) according to one of Claims 1 to 8, for the preparation of a medicament intended for the treatment of contractures, of pain, of Parkinson's disease and of Parkinsonian symptoms, of epilepsy, of mixed forms and other epileptic syndromes as a supplement to another antiepileptic treatment, or in monotherapy, of sleep apnea, and for neuroprotection.

**Patentansprüche**

**1.** Verbindung nach der allgemeinen Formel (I)

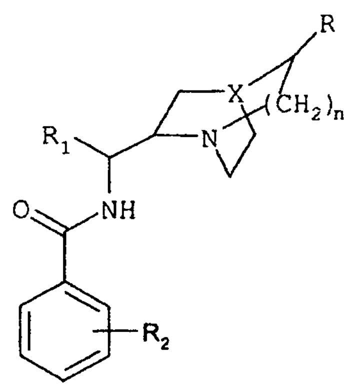

(I)

in welcher

R für ein Wasserstoffatom oder eine Vinylgruppe steht;
n für 0 oder 1 oder 2 steht, wenn R für ein Wasserstoffatom steht, und n für 1 steht, wenn R für eine Vinylgruppe steht;
X für eine Gruppe mit der Formel CH oder für ein Stickstoffatom steht, wenn R für ein Wasserstoffatom steht, und X für eine Gruppe mit der Formel CH steht, wenn R für eine Vinylgruppe steht;
$R_1$ entweder für eine Phenyl- oder Naphthylgruppe steht, die möglicherweise einen oder mehrere Substituenten aufweist, die aus Halogenatomen, geradkettigen oder verzweigten ($C_1$-$C_6$)-Alkyl-, Hydroxy- und ($C_1$-$C_6$)-Alkoxygruppen sowie der Trifluormethylgruppe gewählt sind, oder für eine Cyclohexylgruppe oder für eine Heteroarylgruppe, die aus den Gruppen Thienyl, Pyridinyl, Oxazolyl, Furanyl, Thiazolyl, Chinolinyl und Isochinolinyl gewählt ist;
$R_2$ entweder für ein Wasserstoffatom steht oder für einen oder mehreren Substituenten, die aus Halogenatomen und aus den Gruppen Trifluormethyl, ($C_1$-$C_6$)-Alkyl, ($C_1$-$C_6$)-Alkoxy, Thienyl, Phenyloxy, Hydroxy, Mercapto, Thio-($C_1$-$C_6$)-Alkyl, Cyano oder aus einer Gruppe nach der allgemeinen Formel $-NR_4R_5$, $SO_2NR_4R_5$, $-SO_2$-($C_1$-$C_6$) -Alkyl, $-SO_2$-Phenyl, $-CONR_4R_5$, $-COOR_7$, -CO- ($C_1$-$C_6$) -Alkyl, -CO-Phenyl, $-NHCOR_8$, $-NHSO_2$-($C_1$-$C_6$) -Alkyl, $-NHSO_2$-Phenyl und $-NHSO_2NR_4R_5$ oder aus einer Gruppe mit der Formel $-OCF_2O$-, die an den Positionen 2 und 3 an die Phenylgruppe gebunden ist, gewählt sind;

wobei die Gruppen ($C_1$-$C_6$)-Alkyl, ($C_1$-$C_6$)-Alkoxy, $-SO_2$- ($C_1$-$C_6$) -Alkyl, -CO- ($C_1$-$C_6$) -Alkyl und $-NHSO_2$- ($C_1$-$C_6$)-Alkyl möglicherweise mit einer oder mehreren Gruppen $R_3$ substituiert sein können;
wobei die Gruppen Phenyl, $-SO_2$-Phenyl, -CO-Phenyl und $-NHSO_2$-Phenyl möglicherweise mit einer Gruppe $R_6$ substituiert sein können;

$R_3$ für ein Halogenatom, eine Phenylgruppe, ($C_1$-$C_6$)-Alkoxy, $-NR_4R_5$ steht;
$R_4$ und $R_5$ unabhängig voneinander für ein Wasserstoffatom oder eine ($C_1$-$C_6$)-Alkylgruppe stehen oder $R_4$ und $R_5$ mit dem Stickstoffatom, an welches sie gebunden sind, einen Pyrrolidinring, einen Piperidinring oder einen Morpholinring bilden;
$R_6$ für ein Wasserstoffatom, ein Halogenatom, eine Trifluormethylgruppe, eine Cyanogruppe, eine Hydroxygruppe, eine Mercaptogruppe, eine ($C_1$-$C_6$)-Alkyl- oder eine ($C_1$-$C_6$)-Alkoxygruppe steht;
$R_7$ für ein Wasserstoffatom oder für eine ($C_1$-$C_6$) - Alkylgruppe steht, die möglicherweise mit einer oder mehreren Gruppen $R_3$ substituiert ist, oder für eine Phenylgruppe, die möglicherweise mit einer Gruppe $R_6$ substituiert ist;
$R_8$ für eine ($C_1$-$C_6$)-Alkylgruppe steht, die möglicherweise mit einer oder mehreren Gruppen $R_3$ substituiert ist, oder für eine ($C_1$-$C_6$) - Alkoxygruppe oder für eine Phenylgruppe, die möglicherweise mit einer Gruppe $R_6$ substituiert ist,

in Form der freien Base oder in Form eines Salzes, welches bei Zusatz einer Säure gebildet wird, in Form eines Hydrates oder in Form eines Solvates.

**2.** Verbindung gemäß Anspruch 1, **dadurch gekennzeichnet, dass** sie in Threo-Konfiguration vorliegt.

**3.** Verbindung gemäß einem der Ansprüche 1 und 2, **dadurch gekennzeichnet, dass** n für 0 oder 1 steht.

**4.** Verbindung gemäß Anspruch 3, **dadurch gekennzeichnet, dass** X für eine Gruppe mit der Formel CH steht.

**5.** Verbindung gemäß Anspruch 4, **dadurch gekennzeichnet, dass** R für ein Wasserstoffatom steht.

**6.** Verbindung gemäß Anspruch 5, **dadurch gekennzeichnet, dass** n für 1 steht.

**7.** Verbindung gemäß Anspruch 6, **dadurch gekennzeichnet, dass** $R_1$ für eine Phenylgruppe steht, die möglicherweise substituiert ist.

**8.** Verbindung gemäß Anspruch 1, **dadurch gekennzeichnet, dass** sie aus den folgenden Verbindungen gewählt ist:

Threo-2-chloro-*N*-[(1-azabicyclo[2.2.2]oct-2-yl)phenylmethyl]-3-trifluormethylbenzamid;
Threo-2-chloro-*N*-[(1-azabicyclo[2.2.2]oct-2-yl)phenylmethyl]-3-trifluormethylbenzamid-Chlorhydrat;
Threo-2,6-dichloro-*N*-[(1-azabicyclo[2.2.2]oct-2-yl)phenylmethyl]-3-trifluormethylbenzamid;
Threo-2,6-dichloro-*N*-[(1-azabicyclo[2.2.2]oct-2-yl)phenylmethyl]-3-trifluormethylbenzamid-Chlorhydrat;

2-Chloro-*N*-(8α,9*S*-cinchonan-9-yl)-3-trifluormethylbenzamid;
2-Chloro-*N*-(8α,9*S*-cinchonan-9-yl)-3-trifluormethylbenzamid-Chlorhydrat;
2,6-Dichloro-*N*-[(1*S*)-[(2*S*)(1-azabicyclo[2.2.2]oct-2-yl)phenylmethyl]-3-(trifluormethyl)benzamid;
2,6-Dichloro-*N*-[(1*S*)-[(2*S*)(1-azabicyclo[2.2.2]oct-2-yl)phenylmethyl]-3-(trifluormethyl)benzamid-Chlorhydrat;
Threo-*N*-[1-azabicyclo[2.2.2]oct-2-yl(4-fluorphenyl)methyl]-2,6-dichloro-3-(trifluormethyl)benzamid;
Threo-*N*-[1-azabicyclo[2.2.2]oct-2-yl(4-fluorphenyl)methyl]-2,6-dichloro-3-(trifluormethyl)benzamid-Chlorhydrat;

**9.** Arzneimittel, **dadurch gekennzeichnet, dass** es aus einer Verbindung gemäß einem der Ansprüche 1 bis 8 besteht.

**10.** Pharmazeutische Zusammensetzung, **dadurch gekennzeichnet, dass** sie eine Verbindung gemäß einem der Ansprüche 1 bis 8 sowie mindestens einen pharmazeutisch unbedenklichen Trägerstoff umfasst.

**11.** Verfahren zur Herstellung einer Verbindung gemäß Anspruch 1, **dadurch gekennzeichnet, dass** ein Diamin nach der allgemeinen Formel (II)

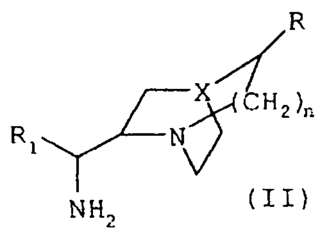

(II)

in welcher n, R und $R_1$ den Begriffsbestimmungen gemäß Anspruch 1 entsprechen, mit einer aktivierten Säure oder mit einer Verbindung nach der allgemeinen Formel (III)

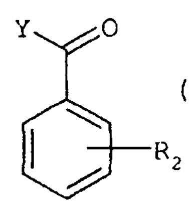

(III)

in welcher Y für eine Abgangsgruppe steht, umgesetzt wird.

**12.** Verfahren zur Herstellung einer Verbindung gemäß Anspruch 1, **dadurch gekennzeichnet, dass** es sich bei der Abgangsgruppe um ein Halogen handelt.

**13.** Verfahren zur Herstellung einer Verbindung gemäß Anspruch 11, **dadurch gekennzeichnet, dass** es eine Verbindung umfasst, die aus den folgenden Verbindungen gewählt ist:

- (Z)-1-Azabicyclo[2.2.2]oct-2-yl(phenyl)methanon-*O*-benzyloxim-Chlorhydrat;
- Threo-[1-azabicyclo[2.2.2]oct-2-yl(phenyl)methyl]amin;
- Threo-2-chloro-N-[(1-azabicyclo[2.2.2]oct-2-yl)phenyl]methyl]amin-Chlorhydrat;
- 8α,9*S*-Cinchonan-9-amin;
- 2-Chloro-*N*-(8α,9*S*-cinchonan-9-yl)-3-trifluormethylbenzamid-Chlorhydrat;
- (1*S*)-[(2*S*)-1-Azabicyclo[2.2.2.]oct-2-yl(phenyl)methyl]amin-D-Tartrat;
- 1-Azabicylo[2.2.2]oct-2-yl(4-fluorphenyl)methanol;
- 1-Azabicylo[2.2.2]oct-2-yl(4-fluorphenyl)methanon;
- (Z)-1-Azabicyclo[2.2.2]octyl(4-fluorphenyl)methanon-O-benzyloxim-Chlorhydrat;
- Threo-1-azabicyclo[2.2.2]oct-yl(4-fluorphenyl)methanamin.

**14.** Verwendung einer Verbindung nach Formel (1) gemäß einem der Ansprüche 1 bis 8 zur Herstellung eines Arzneimittels, welches zur Behandlung von Verhaltensstörungen bestimmt ist, die mit Demenz, Psychosen, verschiedenartigen Formen von Angstzuständen, Panikanfällen, Phobien, Zwangsstörungen, verschiedenen Formen der Depression, Störungen aufgrund von Alkoholmissbrauch oder Alkoholentzug, Störungen des Sexualverhaltens, Ess-Störungen und Migräne in Verbindung stehen.

**15.** Verwendung einer Verbindung nach Formel (I) gemäß einem der Ansprüche 1 bis 8 zur Zubereitung eines Arzneimittels, welches zur Behandlung von krankhaften Verspannungen, von Schmerzen, der Parkinson-Krankheit und parkinsonartigen Symptomen, von Epilepsien, von Mischformen und anderen epileptischen Syndromen, und zwar ergänzend zu einer anderen anti-epileptischen Behandlung oder in Monotherapie, von Schlafapnoe sowie für die Neuroprotektion bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9945011 A
- US 3823134 A
- DE 2436263
- BE 620741
- DE 1158957
- US 3112337 A
- GB 915259 A
- US 3203987 A
- DE 642758
- EP 68700 A
- FR 2396757
- DE 2734270
- US 2273444 A
- DE 19929076
- EP 0556674 A
- US 3663615 A
- DE 2056912
- DE 2901170
- US 3953476 A
- BE 872585
- US 4879426 A
- US 3725417 A
- GB 913100 A
- EP 0556672 A
- US 3801636 A


### Littérature non-brevet citée dans la description


- *Bull.Soc.Chim.Belg.,* 1997, vol. 106, 77-84
- *Tetrahedron : Asymmetry,* 1995, vol. 6, 1699-1702
- *Tetrahedron,* 1999, vol. 55, 2795-2810
- *Chem.Commun.,* 1999, 1927-1928
- *J. Med. Chem.,* 1980, 180-184
- *J. Org. Chem.,* 1985, vol. 50, 29-31
- *Chem. Comm.,* 1999, 1927-1929
- *Arzneim. Forsch.,* 1984, vol. 34 (11a), 1668-1679
- *J. Pharm. Pharmacol.,* 1962, vol. 14, 679-685
- *J. Chem. Soc. (C,* 1968, 13
- *J. Org. Chem.,* 1991, vol. 56 (1), 4976-4977
- *Syn. Lett.,* 1996, 473-474
- *J. Med. Chem.,* 2001, vol. 44, 1085-1098
- *Chem. Pharm. Bull.,* 1988, vol. 36 (9), 3462-3467
- *J. Labelled Compd. Radiopharm.,* 1997, vol. 39 (6), 501-508
- *Tetrahedron Lett.,* 2000, vol. 41, 3157-3160
- *J. Org. Chem.,* 1967, vol. 62 (25), 8634-8639
- *J. Chem. Soc.,* 1927, 25
- *Chem. Pharm. Bull.,* 1992, 1789-1792
- *Aust. J. Chem.,* 1984, 1938-1950
- *J. O. C.,* 1980, 527